# EUROPEAN PATENT APPLICATION

(11) **EP 4 438 078 A1**
(43) Date of publication of application: **02.10.2024**
(21) Application number: 23165704.0
(22) Date of filing: 30.03.2023
(51) Int. Cl.: A61M 5/158, A61M 5/145, A61M 5/142, A61M 39/02, A61M 39/08

(54) **CANNULA ASSEMBLY FOR A WEARABLE DRUG DELIVERY DEVICE**

(71) Applicant: TecMed AG, 3400 Burgdorf (CH)
(72) Inventor: Baumert, Jan, 3455 Grünen (CH); Streit, Ursina, 3422 Kirchberg (CH); Hanimann, Michael, 3012 Bern (CH); Margot, Roland, 3076 Worb (CH); Steiner, Fabian, 3400 Burgdorf (CH); Kobel, Eduard, 3456 Trachselwald (CH)
(74) Representative: Meier Obertüfer, Jürg

(57) **Abstract**

The present invention is concerned with a drug delivery device with a rigid cannula, a soft cannula and an automatic needle or cannula insertion mechanism. A novel design of a cannula assembly uses a sealing sleeve to ensure a fluid-tight slidable connection between the soft cannula and the rigid cannula with improved sealing pressure while allowing damage-free, cost-effective manufacturing.

## Description

### FIELD OF THE INVENTION

The present invention relates to cannula assemblies for use in a drug delivery device, in particular to infusion pumps or injectors with a cannula insertion mechanism. A fluid tight connection between a rigid cannula and a soft cannula is presented providing a design with improved manufacturability at lower cost with optimum reliability.

### BACKGROUND OF THE INVENTION

A variety of diseases exist that require regular treatment by subcutaneous administration of a medicament, and a number of delivery devices have been developed to support a patient in accurately and controllably delivering an amount of drug in a self-administration process. Drug delivery devices include injection devices that are removed from the site of application after each medication event or drug delivery process, as well as infusion devices with a cannula or needle that remains in the skin of the patient for a prolonged period of time. By way of example, diabetes may be treated by administration of insulin by the patients themselves with the help of multi-variable-dose insulin injection pens or infusion pumps. Alternatively, patch injectors, wearable injectors or wearable pumps are patched or adhered to the skin of the patient.

Common to all devices for subcutaneous drug delivery is a reservoir to store the fluid medicament, and a fluid path to bring the drug out of the device and into the subcutaneous tissue of a patient. Many of such devices are designed as patch pumps or patch injectors for attachment to the user's body, where they remain for the duration of injection or infusion as a wearable pump without the need of actively holding the device in place. To further assist the user, patch devices typically include a cannula insertion and retraction mechanism, which allows applying the patch to the skin and removing the device without manual handling of the cannula. The patch device will automatically insert the cannula out of the housing into the body of a patient for delivery of the drug, and also retract the cannula into the housing after an injection or infusion cycle has been terminated. For users' comfort, wearable pumps often use a flexible or soft cannula for drug delivery, which helps users avoiding pain when moving around with a patch pump attached to, and a cannula inserted into the body. A flexible or soft cannula, however, is too soft to pierce the tissue of the body and hence cannot be pushed out of a pump housing and into the tissue of a patient. An extra rigid insertion needle may be needed to support the soft cannula for bringing the soft cannula into delivery position. In a typical design, the rigid cannula is connected to the drug reservoir and provides a fluid connection at the proximal end. The soft cannula is slidingly mounted onto the rigid cannula, with the rigid cannula arranged inside the soft cannula and protruding at the distal end for insertion. After inserting the soft cannula, the rigid insertion needle is retracted into the housing of the patch device, leaving the soft cannula out for drug delivery.

US 6, 960,192 B1 describes such a patch pump with a cannula assembly consisting of a soft cannula and a rigid cannula, where the rigid cannula is arranged inside the soft cannula for insertion, and to slidably move axially back inside the soft cannula for drug delivery. No indication is disclosed of how to realise the sealing function between the soft cannula and the rigid cannula.

EP 3928814 A1 gives more details about the design of a patch pump with an insertion mechanism for a soft cannula by using a rigid cannula. Each of the cannulae is attached to a cannula holder movably mounted relative to the pump housing. Before use, the rigid cannula is slidingly arranged inside the soft cannula, with the tip protruding on the distal end, ready to pierce the skin of a patient. To bring the cannula assembly into a position for drug delivery, an insertion spring is triggered to move both cannula holders and hence both cannulae forward into the body of the patient. Once the soft cannula is inserted, a retraction spring moves the rigid cannula holder and also the rigid cannula back into the housing leaving the soft cannula in the tissue. The medicament can now be transferred from the reservoir through the rigid cannula and the soft cannula into the body of the patient. In this document, the sealing between the soft cannula and the rigid cannula is described with an area of extended wall thickness at the proximal end of the soft cannula, and with a flange to provide a proper grip for the soft cannula when it is moved axially, or when an axial force is applied to the soft cannula, for example when removing the patch pump from the infusion site. While this arrangement may be adequate for a lower range of fluid pressure, it is a weak point when it comes to reliability of drug delivery.

For reliable drug delivery, the entire fluid path from the reservoir to the distal end of the cannula needs to be fluid-tight. As also further explained in EP 3928814 A1, supervision of drug delivery, in particular detection of occlusion, heavily depends on the quality of sealing between the soft cannula and the rigid cannula. To achieve a better sealing is technically linked to applying more sealing pressure, which also makes manufacturing of the needle assembly significantly more difficult because the rigid cannula would possibly damage the inner surface of the soft cannula in the process. A cannula assembly with a weak sealing will typically be easier to manufacture, but will perform worse during drug delivery. Hence, there is clearly a need for a cannula assembly for use in a mobile or wearable drug delivery device which provides a slidable and reliably fluid-tight sealing between the soft cannula and the rigid cannula, while still allowing easy and damage-free manufacturing.

In US 6,960,192 a patch pump with a cannula assembly is disclosed, where the flexible cannula has a sealing portion through which the rigid cannula extends. Just as in EP 3928814 A1, no indication is given of how to technically achieve an optimal sealing function in said sealing portion of the soft cannula.

A common approach to solve manufacturing problems including sealing between different components is to bring components together in an uncompressed or lose state, where assembly of the components can reliably be conducted. A manufacture step is then added to bring components in a compressed state, where pressure between components provide a fluid-tight connection where necessary. A press-fit connection of a plug in an opening of an other component is a very basic example of such a sealing. However, a press-fit connection has very high friction between components and is not suitable for sliding connection of moving components. Alternatively, a thermic shrinkable soft tube with a large diameter may be pulled over a rigid tube or cannula with a small diameter. Heat is applied to shrink the soft tube from a large diameter to a small diameter until it reaches the surface of the rigid tube. This manufacturing process is very easy and can be done without risk of harming the soft tube. However, for medical devices, in particular for components in contact with the drug, materials need to be bio-compatible, restricting the choice of materials. Furthermore, the elasticity of soft tubes is difficult to control with shrinkable tubes, making them less suitable for providing a slidable sealing.

An other common approach to sealingly connect a soft tube to a rigid tube is to add an outer sleeve to the cannula assembly. The outer sleeve may be constructed from a metal or other material which is plastically deformable by mechanical means. When manufacturing the cannula assembly, both soft and rigid cannula would be introduced into the sleeve, and the sleeve would be mechanically deformed by crimping to apply sealing pressure to the outside of the soft cannula. Again, a drawback is introduced by this method, as the deformation of the sleeve may typically not be uniform around the periphery of the soft cannula, and consequently the sealing pressure inside the soft cannula will vary, resulting in a potentially unreliable sealing. Such an outer sleeve, when used to assemble a soft cannula without a flange, may further not be suitable for sealing of sliding components, as the sleeve may slip off the soft cannula during insertion or retraction of the needle assembly. With a thin wall, the soft cannula may also be prone to damage and consequently to leaking.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the drug delivery device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process, whereas the term "infusion system" refers to a device with a cannula or needle that remains in the skin of the patient for a prolonged period of time, for example, several hours.

### DESCRIPTION OF THE INVENTION

It is an objective of the invention to provide a wearable drug delivery device with a cannula insertion mechanism and with an improved cannula assembly, which ensures both damage-free and costeffective manufacturing, and a reliable slidable sealing between a soft cannula and a rigid cannula.

The drug delivery device may be a tubeless patch infusion pump attachable to the skin of a patient by means of an adhesive layer, for subcutaneous delivery of insulin over a period of more than 48 hours through a cannula integrated into the pump. Alternatively, the delivery device is not a tubeless patch infusion pump attachable to the skin of a patient by means of an adhesive layer, for subcutaneous delivery of insulin through a cannula integrated into the pump over a period of more than 48 hours. For instance, the alternative delivery device may be a patch injector attachable to the skin of a patient by means of an adhesive layer, for subcutaneous delivery of a drug other than insulin over a period of less than 48 hours. The alternative delivery device may be a wearable insulin pump or a handheld injection pen devoid of an adhesive layer for attaching to the skin of a patient.

The objective is achieved by introducing a cannula assembly for use in a mobile or wearable drug delivery device, comprising a soft cannula, a rigid cannula, and a sealing sleeve, where the soft cannula comprises a wall forming a soft cannula lumen configured to envelope at least a portion of the rigid cannula and to provide a sliding connection between the rigid and soft cannula. The sealing sleeve is configured to radially envelope at least a portion of the soft cannula. The soft cannula includes a soft cannula flange constructed as a portion with an extended wall thickness at, or near the proximal end of the soft cannula. An inner surface of the sealing sleeve (261) may at least partially engage the outer surface of the soft cannula and may provide a radial sealing pressure between the rigid cannula and the soft cannula, thereby defining a sealing area on the outer surface of the rigid cannula. Said sealing area may be defined as the specific part of the outer surface of the rigid cannula, where the wall of the soft cannula is pressed onto the outer surface of the rigid cannula by a compressing section of the sealing sleeve, providing a sliding sealing between the soft cannula and the rigid cannula.

A mobile or wearable drug delivery device may be realised including the cannula assembly as outlined, where the drug delivery device may be a patch pump or a patch injector or a hand-held injection device.

A method for manufacturing an improved cannula assembly may be used, where the cannula assembly comprises a soft cannula, a rigid cannula, and a sealing sleeve. The soft cannula may comprise a wall forming a soft cannula lumen configured to envelope at least a portion of the rigid cannula, and a soft cannula flange may be constructed as a portion with an extended wall thickness at or near the proximal end of the soft cannula. The sealing sleeve may be configured to envelope at least a portion of the soft cannula. The method may comprise the following steps:
- bringing at least a portion of the rigid cannula into the lumen of the soft cannula
- bringing at least a portion of the soft cannula into the sealing sleeve
- plastically deforming at least a portion of the sealing sleeve to form a compressing section, where the wall of the soft cannula is pressed onto the outer surface of the rigid cannula providing a slidable sealing connection.

A drug delivery device with such an improved cannula assembly allows easy, damage-free and costeffective manufacturing while ensuring optimal control of the sealing pressure and hence the reliability of drug delivery.

### BRIEF DESCRIPTION OF THE DRAWINGS

The subject matter of the invention will be explained in more detail in the following text with reference to preferred exemplary embodiments which are illustrated in the attached drawings, in which:

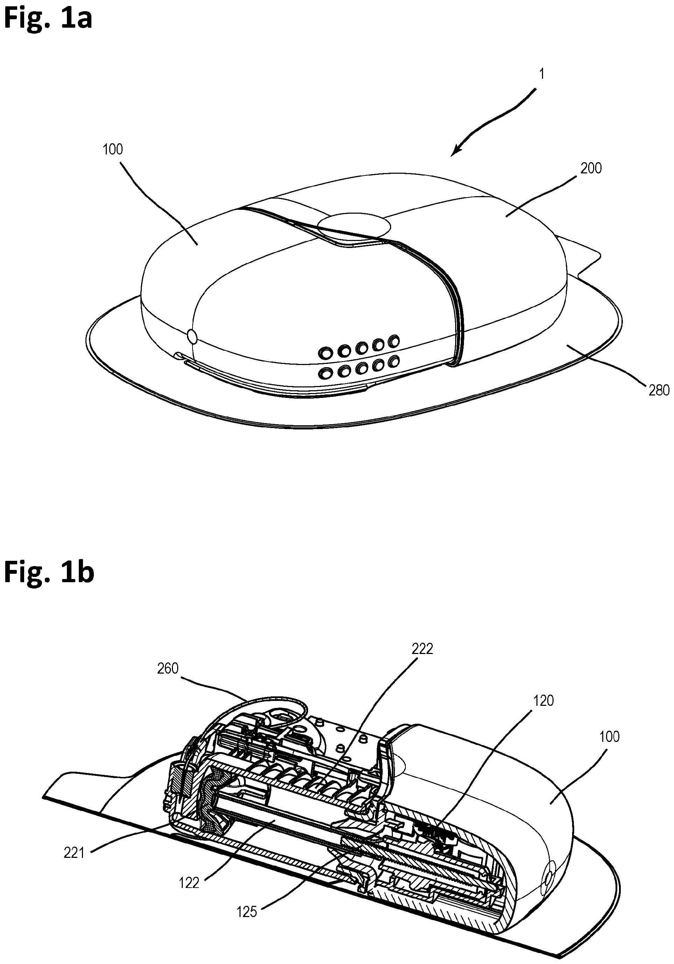

- Fig. 1a, b: depicts a modular patch pump according to the present invention;

- Fig. 1a: depicts a three-dimensional outside view of the patch pump;
- Fig. 1b: depicts a sectional view of the patch pump.
- Fig. 2: depicts the pump unit and the reservoir unit of the patch pump.
- Fig. 3: depicts a view of the pump unit with the housing removed.
- Fig. 4a, b: depicts the reservoir unit with selected parts removed for illustration;

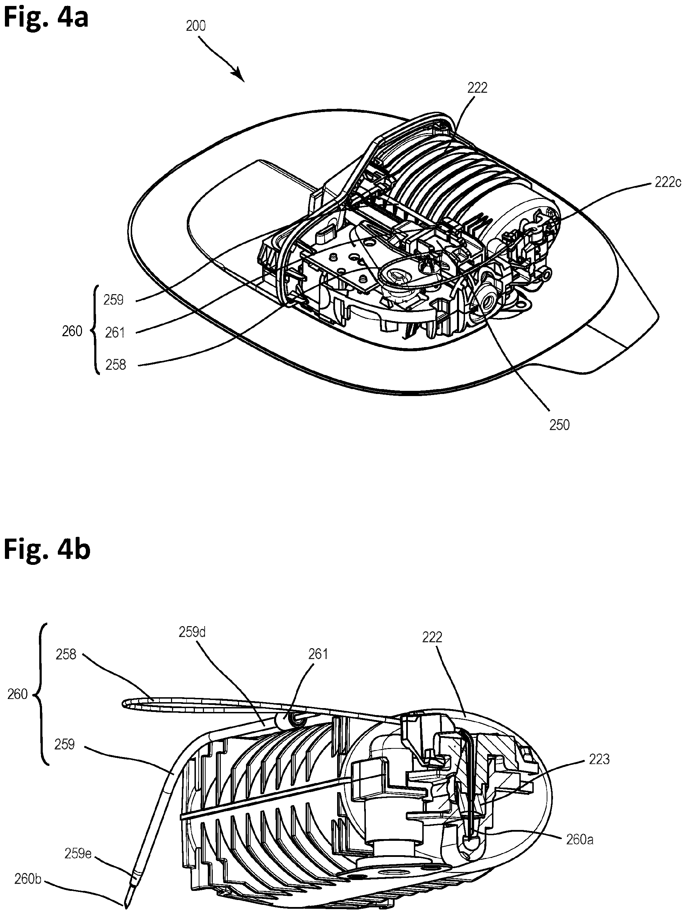

Fig. 4a depicts the reservoir unit with the housing removed;
Fig. 4b depicts an arrangement of the cannula assembly inside the reservoir unit.

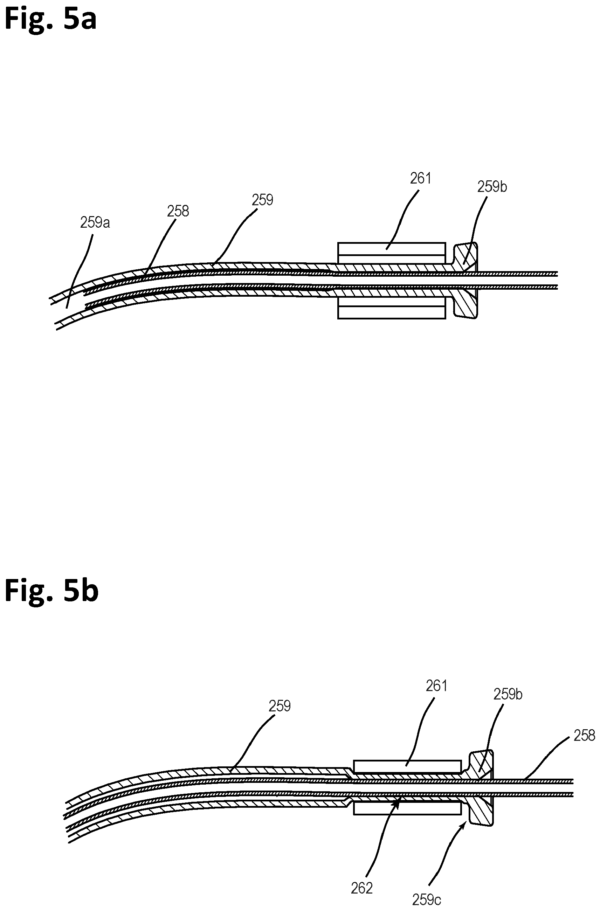

- Fig. 5a, b: depicts a first embodiment of the cannula assembly;
Fig. 5a depicts the first cannula assembly with a sealing sleeve before deformation;
Fig. 5b depicts the first cannula assembly with a sealing sleeve after deformation.

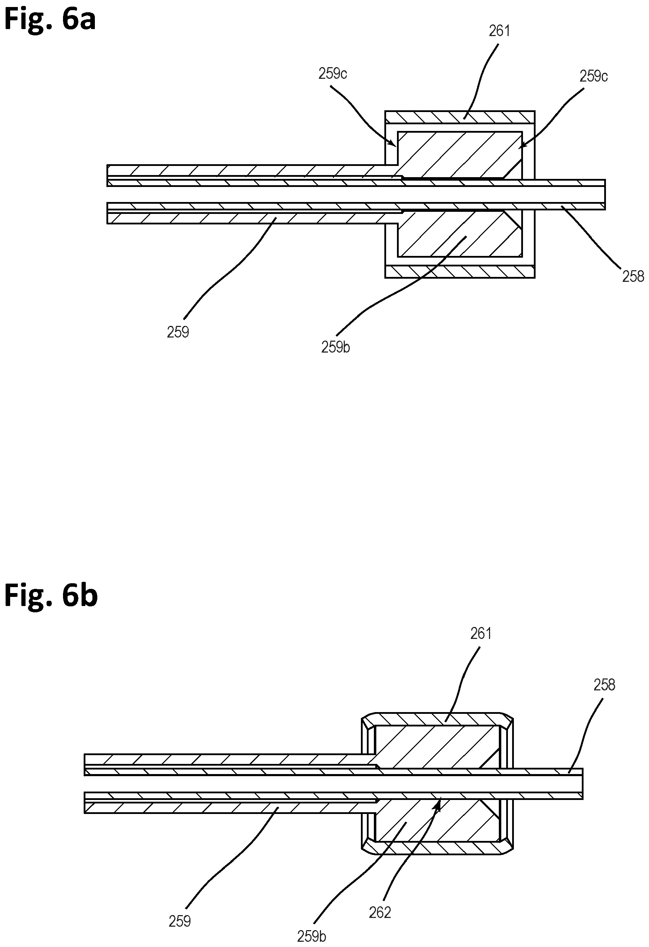

- Fig. 6a, b: depicts a second embodiment of the cannula assembly;
Fig. 6a depicts the second cannula assembly with a sealing sleeve before deformation;
Fig. 6b depicts the second cannula assembly with a sealing sleeve after deformation.

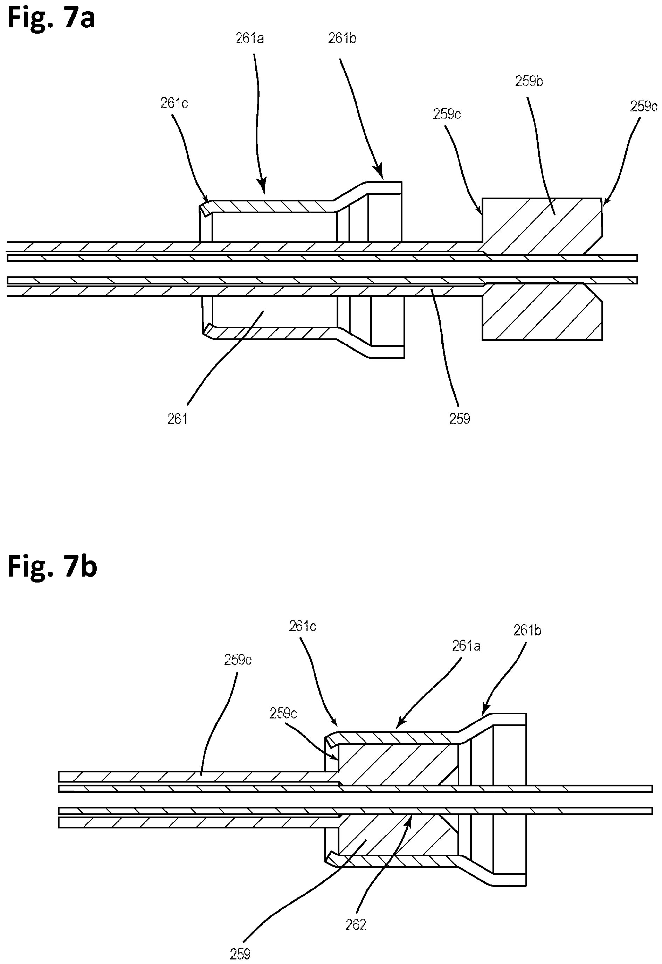

- Fig. 7a, b: depicts a third embodiment of the cannula assembly;
Fig. 7a depicts the third cannula assembly with a sealing sleeve before axial insertion of the sealing flange;
Fig. 7b depicts the third cannula assembly with a sealing sleeve after axial insertion of the sealing flange.

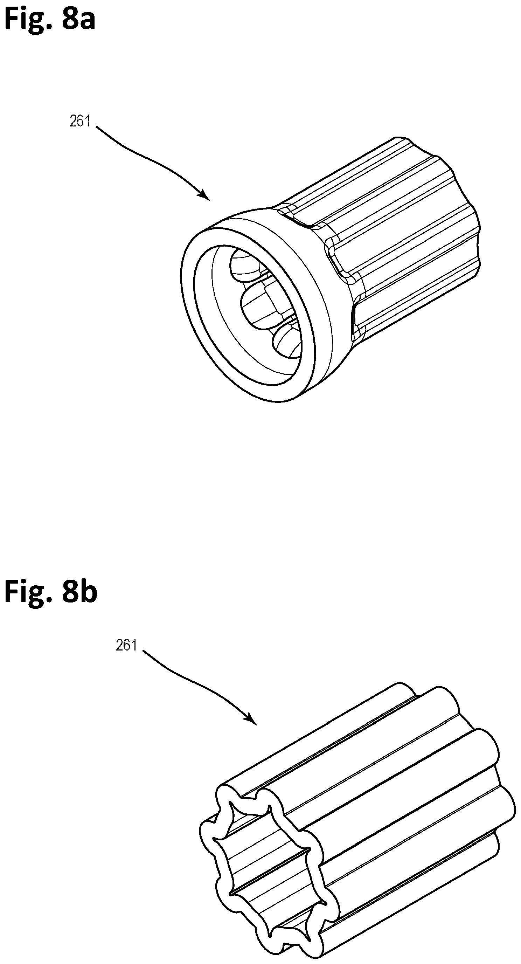

- Fig. 8a, b: depicts examples of shapes for crimping the sealing sleeve;
Fig. 8a depicts a sealing sleeve in a "tulip" shape after incomplete crimping;
Fig. 8b depicts a sealing sleeve after crimping over the complete length.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

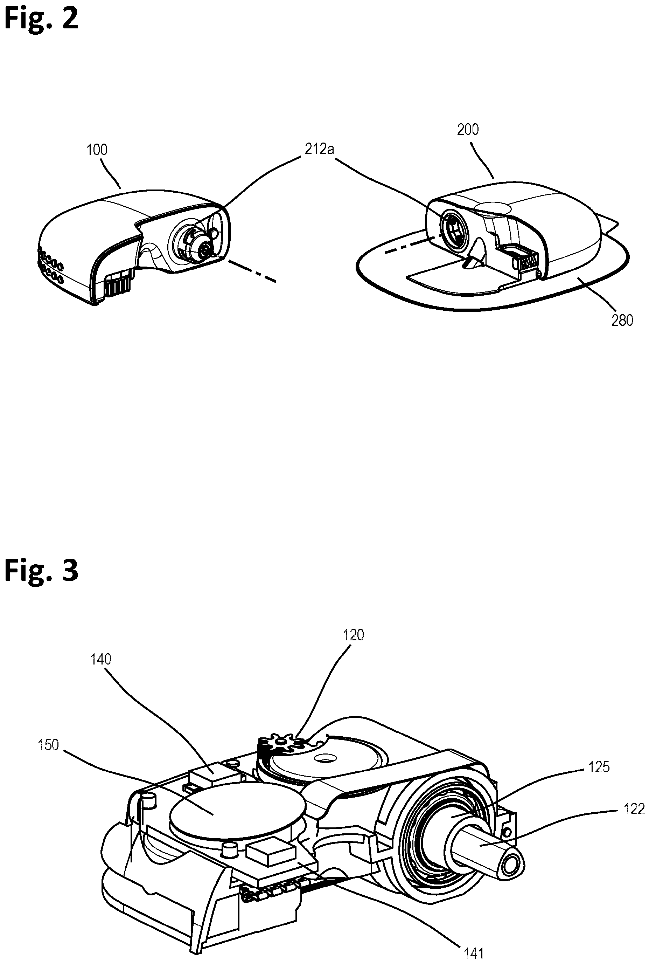

Figure 1a depicts a drug delivery device including a cannula assembly according to the present invention. The drug delivery device is realised as a patch pump (1) attachable to the skin of a patient. The patch pump (1) comprises a reusable pump unit (100) releasably connected via a bayonet connection (212a, see Fig. 2) to a disposable reservoir unit (200). The reservoir unit (200) comprises a reservoir to store the medicament and a needle assembly with a fluid path to bring the drug from the reservoir into the body of the patient. At the bottom of the reservoir unit (200) an adhesive patch assembly (280) is included to attach the patch pump (1) to the body of the patient. The pump unit (100) is releasably and sealingly connected to the reservoir unit (200) by a bayonet connection. Figure 1a shows the complete patch pump (1) with both units joined together, seen from a position above the pump. In the context of the present invention, "above" or "top" refers to the side of the pump which is facing away from the patient's body when the pump is attached to the patient for drug delivery. Consequently,"bottom" or "base" refers to the side of the pump facing towards the patient's body during drug delivery. Figure 1b depicts a cut through the patch pump (1) to show the arrangement of main inner parts of the drug delivery device of figure 1a. The pump unit (100) comprises a drive mechanism (120) for driving the plunger rod (122), an encoder to supervise the movement of the drive mechanism, a rechargeable battery and an control unit configured to control the set-up, drug delivery and supervision of the pump. The battery may be rechargeable and may be configured to be charged by a further battery in the disposable reservoir unit (200) while the drive mechanism (120) is connected to the reservoir unit (200). The drive mechanism (120) is actuated by an electric motor and acts mechanically by transforming the rotation of the motor via a gearbox assembly and a threaded rod (125) to a linear displacement of a plunger rod (122) and from there to a plunger in the reservoir (222) to dispense the medical substance out of the reservoir (222). A gearbox assembly is particularly useful to realise small displacements corresponding to small amounts of drug. The plunger rod may consist of just one threaded rod (125), but alternative embodiments may include a plunger rod assembly consisting of multiple segments While basically every electric motor may be used to drive the drug delivery device, brushless DC or stepper motors are usually preferred for safety reasons... A needle assembly (260) inside the reservoir unit (200) provides the fluid connection from the reservoir (222) to the exterior of the pump for application to the patient. To ensure safe handling, the patch pump (1) is manufactured, shipped, stored and prepared for use with the needle assembly (260) completely inside the enveloping shape of the pump (1). The enveloping shape is an imaginary surface enveloping the housing of the pump (1) while smoothly bridging all gaps and recesses, should any be present, to a closed shell. It is the shape of the pump (1) as perceived by the user from a distance and relevant when it comes to aspects of use like handling or wearability. Preparation of the patch pump in this embodiment includes filling the reservoir inside the disposable reservoir unit (200) from the exterior using a transfer syringe and attaching the pump to the body of the patient by means of the adhesive patch assembly (280). An inserter assembly (250, see Fig. 4a) is included in the disposable reservoir unit (200) and configured to bring an output portion (260b) of the needle assembly (260), in particular the open distal end of the needle assembly (260), out of the enveloping shape of the pump and into the body of the patient once the pump is ready to start drug delivery. In a preferred embodiment of the patch pump (1) the needle assembly (260) includes a rigid cannula (258) and a soft cannula (259), and the inserter assembly (250) is configured to insert the distal end of the soft cannula into the body of the patient using the rigid cannula which will subsequently be retracted for drug delivery.

Figure 2 illustrates the semi-disposable patch pump from Figure 1 with the pump unit (100) and the reservoir unit (200) detached and separated, in a view from above, with the adhesive patch assembly (280) at the bottom towards the body of the patient, and the bayonet connection (212a) in a disengaged state.

Figure 3 further illustrates the pump unit of Figure 1, with selected parts removed to show the inner components. In particular, the drive mechanism (120) is shown with the driving means realised as a threaded rod (125) mounted in, and in cooperation with a plunger rod (122), with the rechargeable battery (150) and the system control circuitry (140) on a printed circuit board (141). The threaded rod (125) and the plunger rod (122) are arranged substantially parallel to the rotation axis of the bayonet connection.

Turning to the reservoir unit (200), Figure 4a gives an overview providing a perspective view with the housing of the reservoir unit removed, in a state before use, when the complete needle assembly (260) is inside the reservoir unit housing, with the proximal end of the rigid cannula (258) connected to the reservoir outlet (222c) and with the soft cannula (259) ready for insertion into the body of a patient. Figure 4a shows the inner parts of the reservoir unit from outside, Figure 4b a similar view with a sectional cut across the outlet of the reservoir (222) to show the design of the rigid cannula (258), with the proximal end of the rigid cannula forming the input portion (260a) of the needle assembly (260). The reservoir outlet sealing (223) ensures a fluid-tight connection between the reservoir (222) and the needle assembly (260). The needle assembly (260) is shown in a retracted position before insertion into the body of a patient. In this position, the entire soft cannula is slidably mounted on the rigid cannula. The tip (260b) of the rigid cannula (260) cannula protrudes at the distal end of the needle assembly, ready for piercing the soft tissue of the patient. The proximal end of the soft cannula (259) is arranged towards the proximal end of the rigid cannula (258). Both the soft cannula (259) and the rigid cannula (258) are operatively connected to the insertion mechanism or inserter assembly (250), which controls the movement of the needle assembly (260) during insertion and retraction, and keeps the components of the needle assembly in a well defined position before, during and after drug delivery. The improved needle assembly (260) includes three main components: a soft cannula (259), a rigid cannula (258) and a sealing sleeve (261). During insertion, all three components are moved forward in a distal direction. With the soft cannula (259) successfully inserted into the body of a patient, the rigid cannula (260) is retracted. Figure 5, Figure 6 and Figure 7 show three different embodiments of such a needle assembly (260). The soft cannula (259) is made of an at least partially elastic material, such as a thermoplastic Fluorinated Ethylene Propylene (FEP) or Polypropylene (PP), and substantially shaped as a tube with a wall surrounding a lumen inside. The rigid cannula is substantially a hollow needle made of a rigid material, such as stainless steel or a rigid plastic. The sliding connection between the rigid cannula (258) and the soft cannula (259) is realised by making the lumen of the soft cannula large enough to allow insertion of the rigid cannula. To achieve a fluid-tight sealing between the two components, a sealing pressure is required, pressing the wall of the soft cannula (259) onto the surface of the rigid cannula (258). These two requirements - easy insertion and fluid-tight sealing - are conflicting and difficult to fulfil in one single mechanical design. The cannula assembly (260) is therefore improved by introducing a sealing sleeve (261), configured to envelope at least a part of the soft cannula. For manufacturing, the sealing sleeve (261) is held in an open state or in a state where the sealing sleeve does not apply a pressure onto the soft cannula. The inner dimension, for example inner diameter of the sealing sleeve is large enough to allow easy insertion of the combined soft and rigid cannula. For use, the sealing sleeve (261) is brought into a position or state where at least a section of the sealing sleeve (261) is radially pressing onto the soft cannula, thereby providing a sealing pressure and establishing a slidable sealing between the rigid cannula (258) and the soft cannula (259). The area on the surface of the rigid cannula where the wall of the soft cannula (259) is pressed onto the rigid cannula shall be termed "sealing area" (262). Figure 5 illustrates a first embodiment of such a cannula assembly, where the sealing sleeve (261) is made of a plastic deformable material, such as aluminium or stainless steel. In an initial state, shown in Figure 5a, the sealing sleeve (261) is provided with an inner diameter large enough to allow the soft cannula and the rigid cannula to be inserted without applying any pressure. In a second step, the sealing sleeve (261) is plastically deformed, for example crimped or swaged onto the soft cannula, which results in a radial sealing pressure from the sealing sleeve via the soft cannula to the outer surface of the rigid cannula. This state is shown in Figure 5b. In this example, the sealing area (262) is next to the soft cannula flange (259b), axially separated, showing that these two features do not need to overlap. A distal stop (259c) makes sure that the soft cannula (259) does not slide backwards when the cannula assembly is pushed in a distal direction for insertion into the tissue of a patient. In this state, the cannula assembly provides a sliding but at the same time reliably fluid-tight sealing, ready for safe and reliable drug delivery and related functions such as occlusion detection, priming of the device or filling of the reservoir.

In the embodiment of Fig. 5, deformation of the sealing sleeve (261) needs to be rather uniform over all its circumference, because the wall of the soft cannula (259) over the sealing area (262) is still thin. Moving the sealing sleeve (261) over the soft cannula flange (259b) provides much more wall thickness that is available for uniform compression in the sealing area (262) and allows for more variation or dimensional tolerance of the inner diameter of the sealing sleeve even in a state for use. The elastic properties of the soft cannula flange (259b) may compensate these dimensional variations and ensure a proper sealing pressure all around the rigid cannula. This is illustrated in Fig. 6, with Fig. 6a showing such an embodiment in a manufacturing state before deformation of the sealing sleeve, and Fig. 6b showing the same embodiment after deformation of the sealing sleeve (261). With the sealing sleeve (261) overlapping the soft cannula flange (259b), both axial ends of the soft cannula flange (259b) may have a proximal or distal stop (259c, Figure 6a) to engage with the deformed ends of the sealing sleeve (261, Figure 6b). This facilitates the fixation of the soft cannula (259) to the inserter assembly (250) while still providing a reliable slidable connection between the soft cannula (259) and the rigid cannula (258). Just like in the example of Fig. 5b, the cannula assembly of Fig. 6b provides a sliding but at the same time reliably fluid-tight sealing.

Fig. 7 illustrates that the concept of combining a soft cannula flange with a sealing sleeve is not restricted to examples where the sealing sleeve is deformed during manufacturing. In this example, the sealing sleeve (261) has a substantially cylindrical shape with two diameters and a gradual decrease from a larger diameter in an entrance section (261b) to a compressing section (261a). At the distal end of the sealing sleeve (261), a second reduction of diameter, a sleeve stop (261c) or an end wall may be provided. At the proximal end of an entrance section (261b), the inner diameter is configured large enough to receive the soft cannula and the rigid cannula for damage-free insertion, typically a 0.1 to 1mm larger than the outer diameter of the soft cannula flange in a relaxed, uncompressed state. During manufacturing, the rigid cannula is first inserted into the lumen of the soft cannula. The two cannulae are then axially inserted together into the inner volume of the sealing sleeve. This is the state shown in Fig. 7a. The insertion will typically be continued by pushing the soft cannula at the proximal stop (259c) of the soft cannula (259) in the distal direction into the open entrance section (261b) of the sealing flange (261), and further into the compression section (261a) until a sufficient length of the soft cannula flange (259b) is compressed, typically until the distal stop (259c) of the soft cannula flange (259) reaches the sleeve stop (261c). This is the state shown in Fig. 7b. Pressing the soft cannula flange (259) into the compressing section (261a) of the sealing sleeve (261) provides a radial sealing pressure with thereby forming sealing area(262) without deforming the sealing sleeve (261). As the compressing section (261a) may itself be of a cylindrical shape, compressing pressure will typically be substantially uniform over the complete circumference of the soft cannula flange, which may further improve the fluid-tight sealing between the soft cannula (259) and the rigid cannula (258). The proximal and/or distal stop (259c) improves fixation of the soft cannula (259) to the inserter assembly (250) and ensures a sliding but at the same time reliably fluid-tight sealing. The order of manufacturing steps as described may be chosen differently, and combinations of steps may be chosen - such as pre-crimping a sealing sleeve to a shape according to Fig. 7a before inserting the cannulae, or applying a crimping step after completing assembly as shown in Fig. 7b. Many other examples are possible by adding a soft cannula flange to a soft cannula, and by adding a sealing sleeve to provide a sealing pressure, without leaving the scope of the invention as claimed.

An other way to implement variations of the current invention is to vary the form of deformation of the sealing sleeve (261). A process called swaging may be applied instead of crimping, resulting in a more uniform deformation similar to the shape shown in Fig. 7, with the same improving effect on uniformity of sealing pressure. When it comes to crimping the sealing sleeve, any deformation is possible which reduces the inner dimensions of the sealing sleeve and thereby creates a sealing pressure. It is a direct effect of the increased wall thickness of the soft cannula flange (259b) with the reduced inner dimension of the sealing sleeve (262) that the shape of deformation is less of an issue, because the elasticity of the soft cannula flange will help ensuring that a minimal pressure is provided over the sealing area completely covering the circumference of the rigid cannula. Further, crimping may not be applied over the complete length of the sealing sleeve, but only on a specific compressing section. Fig. 8 illustrates two possible shapes for crimping the sealing sleeve: crimping an axial section of the sealing sleeve may result in a "tulip" shape, as shown in Fig. 8a, and a complete crimping over the full length of the sealing sleeve in Fig. 8b. Crimping a section of the sealing sleeve may improve the axial fixation of the soft cannula with respect to the inserter assembly (250), just like the proximal or distal stop of the soft cannula flange described before. Further manufacturing steps may be included such as measuring or monitoring the tightness of the seal between the soft cannula and the rigid cannula during crimping such that each sealing sleeve is individually and axially crimped up to the level for reaching an uniform tightness of the seal.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

### LIST OF REFERENCE NUMERALS

- 1: Patch pump
- 100: Pump unit
- 120: Drive mechanism
- 122: Plunger rod
- 125: Threaded rod
- 140: System control circuitry
- 141: Printed circuit board, PCB-PU
- 150: Rechargeable battery
- 200: Reservoir unit
- 212a: Bayonet connection
- 221: Plunger
- 222: Reservoir
- 222c: Reservoir outlet
- 223: Reservoir outlet sealing
- 250: Inserter assembly
- 258: Rigid cannula
- 259: Soft cannula
- 259a: Soft cannula lumen
- 259b: Soft cannula flange
- 259c: Proximal or distal stop
- 259d: proximal end of soft cannula
- 259e: distal end of soft cannula, output portion of the needle assembly
- 260: Cannula assembly or needle assembly
- 260a: Input portion
- 260b: Output portion
- 261: Sealing sleeve
- 261a: compressing section
- 261b: entrance section
- 261c: sleeve stop
- 262: Sealing area
- 280: Adhesive patch assembly

## Claims

1. A cannula assembly (260) for use in a mobile or wearable drug delivery device (1), comprising a soft cannula (259), a rigid cannula (258), and a sealing sleeve (261),
whereby the soft cannula (259) comprises a wall forming a soft cannula lumen (259a) configured to envelope at least a portion of the rigid cannula (258) and to provide a sliding connection; and whereby the sealing sleeve (261) is configured to radially envelope at least a portion of the soft cannula (259);
***characterised by***
the soft cannula (259) includes a soft cannula flange (259b) constructed as a portion with an extended wall thickness at or near a proximal end of the soft cannula (259); and
an inner surface of the sealing sleeve (261) at least partially engages the outer surface of the soft cannula (259) and provides a radial sealing pressure between the rigid cannula (258) and the soft cannula (259), thereby defining a sealing area (262) on the outer surface of the rigid cannula (258).

2. The cannula assembly (260) according to claim 1 whereby the soft cannula flange (259b) includes a proximal or a distal stop (259c) configured to engage a sleeve stop (261c) of the sealing sleeve (261).

3. The cannula assembly (260) according to claim 1 or 2 whereby the soft cannula flange (259b) is configured to at least partially cover the sealing area (262).

4. The cannula assembly (260) according to any of claim 1 to 3 whereby the soft cannula flange (259b) comprises an elastic deformable material.

5. The cannula assembly (260) according to claim 4 whereby the sealing sleeve (261) comprises a compressing section (261a) configured to receive the cannula flange (259b) in an elastic deformed or compressed state.

6. The cannula assembly (260) according to claim 4 whereby the sealing sleeve (261) comprises an entrance section (261b) configured to receive the cannula flange (259b) in an elastic undeformed or uncompressed state.

7. The cannula assembly (260) according to any preceding claim whereby the sealing sleeve (261) is constructed from a plastic deformable material such as a metal.

8. The cannula assembly (260) according to claim 7 whereby the compressing section (261a) of the sealing sleeve (261) is in a deformed state.

9. The cannula assembly (260) according to claim 7 whereby at least a portion of the sealing sleeve (261) is crimped or swaged onto the soft cannula (259).

10. The cannula assembly (260) according to any preceding claim, whereby the rigid cannula (258) includes a 5-face cutting at the distal end to support damage-free insertion of the rigid cannula (258) into the soft cannula (259).

11. A mobile or wearable drug delivery device (1) comprising the cannula assembly (260) according to claims 1 to 10 whereby the drug delivery device (1) is a patch pump or a patch injector.

12. A method for manufacturing a cannula assembly (260) for a mobile or wearable drug delivery device (1), the cannula assembly (260) comprising a soft cannula (259), a rigid cannula (258), and a sealing sleeve (261);
whereby the soft cannula (259) comprises a wall forming a soft cannula lumen (259a) configured to envelope at least a portion of the rigid cannula (258), and a soft cannula flange (259b) constructed as a portion with an extended wall thickness at or near the proximal end (259d) of the soft cannula (259);
and whereby the sealing sleeve (261) is configured to envelope at least a portion of the soft cannula (259);
the method comprising the steps of
- bringing at least a portion of the rigid cannula (258) into the lumen of the soft cannula (259a)
- bringing at least a portion of the soft cannula (259) into the sealing sleeve (261)
- plastically deforming at least a portion of the sealing sleeve (261) to form a compressing section (261a), where the wall of the soft cannula (259) is pressed onto the outer surface of the rigid cannula (258) providing a slidable sealing connection.

13. The method according to claim 12 whereby the soft cannula flange (259b) includes a distal or a proximal stop (259c) configured to engage the sealing sleeve (261).

14. The method according to claim 12 or 13 whereby the soft cannula flange (259b) is configured to at least partially cover the sealing area (262).

15. The method according to any of claim 11 to 14 whereby the soft cannula flange (259b) is constructed from of an elastic deformable material.
